# EUROPÄISCHE PATENTSCHRIFT

(11) **EP 2 099 522 B1**
(45) Veröffentlichungstag und Bekanntmachung des Hinweises auf die Patenterteilung: **13.04.2011**
(21) Anmeldenummer: 07856182.6
(22) Anmeldetag: 16.11.2007
(51) Int. Cl.: A61N 5/06

(54) **BRÄUNUNGSGERÄT ODER STRAHLERMODUL FÜR KOSMETISCHE HAUTBEHANDLUNG**
TANNING APPLIANCE OR RADIATOR MODULE FOR COSMETIC SKIN TREATMENT
APPAREIL DE BRONZAGE OU MODULE RAYONNANT POUR TRAITEMENT COSMÉTIQUE DE LA PEAU

(30) Priorität: 08.12.2006 DE 102006058279
(43) Veröffentlichungstag der Anmeldung: 16.09.2009
(73) Patentinhaber: Heraeus Noblelight GmbH, 63450 Hanau (DE)
(72) Erfinder: BERGER, Ulrich, 63599 Biebergemuend-Breitenborn (DE)
(74) Vertreter: Kühn, Hans-Christian
(86) Internationale Anmeldenummer: PCT/EP2007/009928
(87) Internationale Veröffentlichungsnummer: WO 2008/067902

(56) Entgegenhaltungen:
- WO-A-2005/086846
- WO-A-2006/113577
- DE-A1- 4 324 007
- DE-A1- 19 543 167
- DE-B1- 2 848 234

## Beschreibung

Die vorliegende Erfindung betrifft ein Bräunungsgerät oder Strahlermodul für kosmetische Hautbehandlung.

Derartige Module enthalten gemäß DE 201 17 228 U1 einen Bräunungsstrahler mit ein bis zwei scheibenförmigen Strahlungsfiltern zum Abfiltern des Spektrums. Hierfür überdeckt ein Strahlungsfilter die Strahlungsaustrittsfläche eines Reflektors.

DE 101 51 850 beschreibt ein Bräunungsmodul mit Bräunungsstrahlern mit einer elektrischen Leistung im Bereich von 250 bis 1.000 Watt, einem Reflektorraum und einem Strahlungsfilter.

DE 195 43 167 beschreibt eine Quecksilber (Hg)-Metallhalogenid-Hochdruck-Entladungslampe zur Körperbestrahlung mit lang anhaltender Tiefenbräunung. Diese Entladungslampe weist einen Quarzglaskolben als Leuchtrohr auf, dessen Durchmesser kleiner ist als der Abstand der in diesem Kolben angeordneten Elektroden.

Es ist die Aufgabe der vorliegenden Erfindung, ein vereinfachtes Bräunungsgerät oder ein vereinfachtes Strahlermodul für die kosmetische Anwendung bereit zu stellen und die Strahlung für die Bräunung optimaler zu dosieren.

Die Lösung der Aufgabe erfolgt mit den Merkmalen der unabhängigen Ansprüche. In den abhängigen Ansprüchen sind bevorzugte Ausführungen beschrieben.

Maßgeblich für die vorliegende Erfindung ist, dass zwischen dem Leuchtrohr und der Schutzscheibe keine Filterscheibe mehr nötig ist. Weiterhin ist das Leuchtrohr mit Vanadium (V) und gegebenenfalls Cer (Ce) oder Titan (Ti) dotiert oder mit Ce und Ti.

In einer alternativen Ausführung mit Filterscheibe liegt die Transmissionskante der Filterscheibe bei niedrigeren Wellenlängen als die des Leuchtrohres. Maßgeblich hierfür ist der Betriebszustand. Bewährt hat sich eine Ausführung mit einer Lampe, deren Transmissionskante sich vom ausgeschalteten Zustand zum Betriebszustand zwischen 20 und 50 nm zu höheren Wellenlängen verschiebt. Hierzu eignen sich insbesondere Hüllrohre mit einer Transmissionskante von über 250 nm, die gegebenenfalls bis zu 350 nm im Betriebszustand erreichen.

Filterscheiben sind beschichtete oder spezielle Gläser mit definiertem Transmissionsbereich.

Erfindungsgemäße Bräunungsgeräte oder Strahlermodule für kosmetische Hautbehandlung sind mit einer Schutzscheibe ausgestattet. Eine Schutzscheibe verursacht bedeutend weniger Aufwand als eine Filterscheibe. Bevorzugt werden Acryl- oder Quarz-Schutzscheiben angewendet.

Bestehende Bräunungsgeräte oder Strahlermodule für kosmetische Hautbehandlung können erfindungsgemäß umgerüstet werden, indem die bisher verwendeten Hg-Metallhalogenid-Hochdruck-Entladungslampen ausgetauscht werden durch derartige Lampen mit einem Leuchtrohr, welches mindestens 99,5 Gew.-% Si02 aufweist und mit Vanadium und gegebenenfalls Ce oder mit Ce und Ti dotiert ist.

Nicht geeignete Filterscheiben sind zu entfernen oder gegebenenfalls durch Filterscheiben zu ersetzen, deren Transmissionskante bei niedrigeren Wellenlängen liegt als die Transmissionskante des Leuchtrohres.

Die Verwendung von Hg-Metallhalogenid-Hochdruck-Entladungslampen, deren Elektrodenabstand größer ist als der innere Leuchtrohr-Durchmesser und deren Leuchtrohr mindestens 99,5 Gew.-% Si02 aufweist und erfindungsgemäß mit V und gegebenenfalls Ce oder mit Ce und Ti dotiert ist, ermöglicht das Weglassen der Filterscheibe bei gleichzeitig optimierter Strahlungsdosierung eines Bräunungsgerätes oder Strahlermoduls für kosmetische Hautbehandlung.

Hg-Hochdrucklampen (High pressure lamps) weisen einen Betriebsdruck von 3 bis 10 bar auf und sind wandstabilisiert.

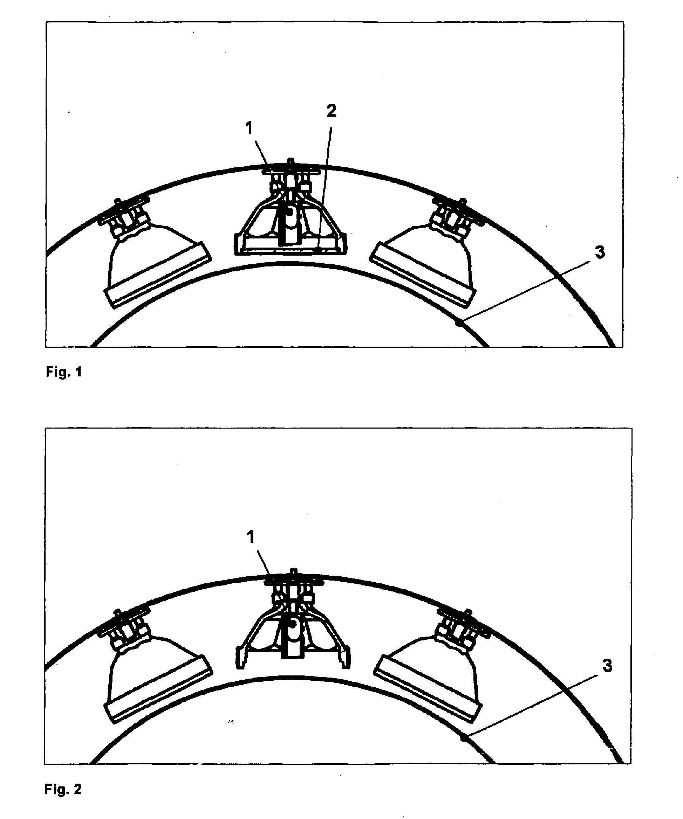

Im Folgenden wird die Erfindung anhand von Beispielen mit Bezug auf Figuren 1 und 2 verdeutlicht.
- Fig. 1: zeigt ein typisches Bräunungsgerät mit einem UV-Strahler, einer Filterscheibe und einer Schutzscheibe.
- Fig. 2: zeigt ein Bräunungsgerät mit einem UV-Strahler mit einer Schutzscheibe, aber ohne Filterscheibe.

Das in Fig. 1 gezeigte Bräunungsgerät weist eine Hg-Metallhalogenid-Hochdruck-Entladungslampe (UV-Lampe) 1, eine Filterscheibe 2, sowie eine Schutzscheibe 3 auf, wobei die UV-Intensität durch die Wahl einer geeigneten Transmissionskante der Filterscheibe 2 von deren Material abhängig eingestellt wird. Zweckmäßigerweise liegt die Transmissionskante der Filterscheibe bei höheren Wellenlängen als die Transmissionskante des Leuchtrohres der UV-Lampe 1. Damit regelt die Filterscheibe die UV-Intensität, respektive die erythemwirksame Bestrahlungsstärke. Nachteil dieses Verfahrens ist, dass die Transmissionskante der Filterscheibe 2 auch temperaturabhängig ist und nur sehr schwer durch entsprechende Kühlung beeinflusst werden kann. Die Schutzscheibe 3 dient als Berührungsschutz für den Endverbraucher.

Erfindungsgemäß besteht der UV-Strahler 1 aus Fig. 1 aus einem Leuchtrohr aus reinem Quarzglas, welches lediglich mit V und gegebenenfalls mit Ce oder Ti dotiert ist. Alternativ kann die Dotierung auch aus Ce und Ti bestehen. Dabei weist die Filterscheibe 2 eine Transmissionskante bei einer niedrigeren Wellenlänge auf als die Transmissionskante des Leuchtrohres der UV-Lampe 1. Damit werden die UV-Intensität, respektive die erythemwirksame Bestrahlungsstärke im Wesentlichen durch das Leuchtrohr der UV-Lampe definiert. Die UV-Intensität, respektive die erythemwirksame Bestrahlungsstärke werden erfindungsgemäß auf die typische Betriebstemperatur der UV-Lampe eingestellt. Somit werden die Schwankungen der UV-Intensität vermieden.

Fig. 2 zeigt ein erfindungsgemäßes Bräunungsgerät oder Strahlermodul für kosmetische Hautbehandlung ohne Filterscheibe, wobei die UV-Intensität allein durch die Wahl einer geeigneten Transmissionskante des Leuchtrohres der UV-Lampe 1 eingestellt ist. Somit ist das Leuchtrohr in dieser Ausführung das einzige Bauteil, mit dem die UV-Intensität, respektive die erythemwirksame Bestrahlungsstärke, eingestellt ist. Das Einsparen der Filterscheibe senkt sowohl Aufwand als auch Kosten. Weiterhin wird der Strahlerwechsel vereinfacht. Von erheblichem Vorteil ist, dass eine Bruchsicherung für die Filterscheibe obsolet wird. Die Schutzscheibe 3 dient weiterhin als Berührungsschutz für den Endverbraucher.

## Patentansprüche

1. Bräunungsgerät oder Strahlermoduf für kosmetische Hautbehandlung,
enthaltend eine Schutzscheibe und eine Hg-Metallhalogenid-Hochdruck-Entladungslampe, deren Elektrodenabstand größer ist als der innere Leuchtrohr-Durchmesser, wobei
das Leuchtrohr mindestens 99,5 Gew.-% SiO₂ aufweist,
**dadurch gekennzeichnet, dass**
zwischen dem Leuchtrohr und der Schutzscheibe keine Filterscheibe angeordnet ist und das Leuchtrohr mit V dotiert ist,
und das Leuchtrohr eine Transmissionskante von über 250 nm und bis zu 350 nm im Betriebszustand aufweist.

2. Bräunungsgerät oder Strahlermodul für kosmetische Hautbehandlung,
enthaltend eine Schutzscheibe, eine Filterscheibe und eine Hg-Metallhalogenid-Hochdruck-Entladungslampe, deren Elektrodenabstand größer ist als der innere Leuchtrohr-Durchmesser,
wobei das Leuchtrohr mindestens 99,5 Gew.-% SiO₂ aufweist,
**dadurch gekennzeichnet, dass**
das Leuchtrohr mit V dotiert ist und
die Transmissionskante der Filterscheibe im Betriebszustand bei niedrigeren Wellenlängen liegt als die Transmissionskante des Leuchtrohres und
im ausgeschalteten Zustand die Transmissionskante der Filterscheibe bei höheren Wellenlängen liegt als die Transmissionskante des Leuchtrohres.

3. Verwendung einer Hg-Metallhalogenid-Hochdruck-Entladungslampe, deren Elektrodenabstand größer ist als der innere Leuchtrohr-Durchmesser und deren Leuchtrohr mindestens 99,5 Gew.-% SiO₂ aufweist und deren Leuchtrohr mit V dotiert ist, in einem Bräunungsgerät oder Strahler für kosmetische Hautbehandlung wobei das Leuchtrohr eine Transmissionskante von über 250 nm und bis zu 350 nm im Betriebszustand aufweist.

4. Verwendung einer Hg-Metallhalogenid-Hochdruck-Entladungslampe, deren Elektrodenabstand größer ist als der innere Leuchtrohr-Durchmesser und deren Leuchtrohr mindestens 99,5 Gew.-% SiO₂ aufweist und deren Leuchtrohr mit V dotiert ist, in einem Bräunungsgerät oder Strahlermodul für kosmetische Hautbehandlung, wobei das Bräunungsgerät oder Strahlermodul für kosmetische Hautbehandlung eine Filterscheibe aufweist, die Transmissionskante der Filterscheibe im Betriebszustand bei niedrigeren Wellenlängen liegt als die Transmissionskante des Leuchtrohres und im ausgeschalteten Zustand die Transmissionskante der Filterscheibe bei höheren Wellenlängen liegt als die Transmissionskante des Leuchtrohres.

## Claims

1. A tanning apparatus or radiator module for cosmetic skin treatment,
comprising a safety screen and an Hg metal halide high-pressure discharge lamp the electrode distance of which is greater than the inner diameter of the illuminating tube, wherein
the illuminating tube incorporates at least 99.5 per cent by weight of SiO₂,
**characterized in that**
there is no filter disc arranged between the illuminating tube and the safety screen
and the illuminating tube is doped with V,
and the illuminating tube incorporates a transmission edge of more than 250 nm and up to 350 nm in the operating state.

2. A tanning apparatus or radiator module for cosmetic skin treatment,
comprising a safety screen, a filter disc and an Hg metal halide high-pressure discharge lamp the electrode distance of which is greater than the inner diameter of the illuminating tube,
wherein the illuminating tube incorporates at least 99.5 per cent by weight of SiO₂,
**characterized in that**
the illuminating tube is doped with V and,
in the operating state, the transmission edge of the filter disc is positioned at lower wavelengths than the transmission edge of the illuminating tube and,
in the switched-off state, the transmission edge of the filter disc is positioned at higher wavelengths than the transmission edge of the illuminating tube.

3. Use of an Hg metal halide high-pressure discharge lamp the electrode distance of which is greater than the inner diameter of the illuminating tube and the illuminating tube of which incorporates at least 99.5 per cent by weight of SiO₂ and the illuminating tube of which is doped with V, in a tanning apparatus or radiator for cosmetic skin treatment, wherein the illuminating tube incorporates a transmission edge of more than 250 nm and up to 350 nm in the operating state.

4. Use of an Hg metal halide high-pressure discharge lamp the electrode distance of which is greater than the inner diameter of the illuminating tube and the illuminating tube of which incorporates at least 99.5 per cent by weight of SiO₂ and the illuminating tube of which is doped with V, in a tanning apparatus or radiator module for cosmetic skin treatment, wherein the tanning apparatus or radiator module for cosmetic skin treatment incorporates a filter disc, wherein, in the operating state, the transmission edge of the filter disc is positioned at lower wavelengths than the transmission edge of the illuminating tube and wherein, in the switched-off state, the transmission edge of the filter disc is positioned at higher wavelengths than the transmission edge of the illuminating tube.

## Revendications

1. Appareil de bronzage ou module de lampe pour le traitement cosmétique de la peau,
contenant un disque protecteur et une lampe à décharge haute pression à halogénure de métal de Hg dont l'écart entre les électrodes est supérieur au diamètre interne du tube lumineux, dans lequel
le tube lumineux présente au moins 99,5 % en poids de SiO₂,
**caractérisé en ce que**
aucun disque de filtration n'est disposé entre le tube lumineux et le disque protecteur
et le tube lumineux est dopé de V,
et le tube lumineux présente une arête de transmission de plus de 250 nm et jusqu'à 350 nm à l'état de marche.

2. Appareil de bronzage ou module de lampe pour le traitement cosmétique de la peau,
contenant un disque protecteur, un disque de filtration et une lampe à décharge haute pression à halogénure de métal de Hg dont l'écart entre les électrodes est supérieur au diamètre interne du tube lumineux,
dans lequel le tube lumineux présente au moins 99,5 % en poids de SiO₂,
**caractérisé en ce que**
le tube lumineux est dopé de V et
l'arête de transmission du disque de filtration se situe à des longueurs d'onde inférieures à l'état de marche que l'arête de transmission du tube lumineux et
l'arête de transmission du disque de filtration se situe à des longueurs d'onde supérieures à l'état éteint que l'arête de transmission du tube lumineux.

3. Utilisation d'une lampe à décharge haute pression à halogénure de métal de Hg dont l'écart entre les électrodes est supérieur au diamètre interne du tube lumineux et dont le tube lumineux présente au moins 99,5 % en poids de SiO₂ et dont le tube lumineux est dopé de V, dans un appareil de bronzage ou une lampe pour le traitement cosmétique de la peau, dans laquelle le tube lumineux présente une arête de transmission de plus de 250 nm et jusqu'à 350 nm à l'état de marche.

4. Utilisation d'une lampe à décharge haute pression à halogénure de métal de Hg dont l'écart entre les électrodes est supérieur au diamètre interne du tube lumineux et dont le tube lumineux présente au moins 99,5 % en poids de SiO₂ et dont le tube lumineux est dopé de V, dans un appareil de bronzage ou module de lampe pour le traitement cosmétique de la peau, dans laquelle l'appareil de bronzage ou le module de lampe pour le traitement cosmétique de la peau présente un disque de filtration, l'arête de transmission du disque de filtration se situe à des longueurs d'onde inférieures à l'état de marche que l'arête de transmission du tube lumineux et l'arête de transmission du disque de filtration se situe à des longueurs d'onde supérieures à l'état éteint que l'arête de transmission du tube
lumineux.
